# EUROPEAN PATENT SPECIFICATION

(11) **EP 1 536 016 B1**
(45) Date of publication and mention of the grant of the patent: **23.08.2006**
(21) Application number: 04023430.4
(22) Date of filing: 01.10.2004
(51) Int. Cl.: C12P 7/06

(54) **Improved process for the preparation of ethanol from cereals**
Verbessertes Verfahren zur Herstellung von Äthanol aus Getreide
Procédé de préparation améliorée d'éthanol à partir de céréales

(30) Priority: 07.10.2003 IT MI20031926
(43) Date of publication of application: 01.06.2005
(73) Proprietor: Etea S.r.l., 12038 Savigliano (CN) (IT)
(72) Inventor: Frandino, Mario, 12036 Revello (CN) (IT)
(74) Representative: Pipparelli, Claudio

(56) References cited:
- EP-A- 0 237 520
- WO-A-02/074895
- FR-A- 2 609 046
- GB-A- 2 091 293

## Description

The present invention relates to an improved process for the production of ethanol from cereals, in which the drying of the non-fermentable residues after distillation is effected using overheated vapour, the ground product from the mill is mixed only with the condensates returning from the process, and the fluid mix of hydrolyzed starch sent to the saccharification and fermentation units is diluted with the distillation residues: this process allows the production of ethanol according to the best transformation yields and with the best possible quality, at the same time, reducing the specific energy consumption per liter of ethanol produced, as well as the quantity of the various ingredients necessary for the production.

It is well known that there are several raw materials which can be involved in the preparation of ethanol, starting from fruit musts, sugary liquids, to cellulose and starchy materials. When the latter are used as raw materials, cereals (corn, rye, barley, wheat) have the greatest importance: starting from these substances, the preparation process includes, before the distillation step, the starch hydrolysis, saccharification and fermentation steps.

Hydrolysis-saccharification consists in the transformation of starch into dextrose, through a reaction catalysed by particular enzymes and physico-chemical conditions.

The solution, after saccharification, is sent for fermentation for the preparation of alcoholic wine.

This operation is normally carried out inside large steel shafts, cooled by means of heat exchangers and/or water coils, after the addition of saccharomyces yeasts which effect the transformation of the simple sugars into ethanol and carbon dioxide.

Distillation represents the last step in the preparation of ethanol, and has the purpose of separating alcohol from all the other components present in wine: insoluble and soluble solids, fats, salts, proteins, water and other volatile matter which is formed during fermentation.

Technologies relating to the ethanol production process have been well known and consolidated for a long time and are constantly monitored with the aim of producing a high quality alcohol at a low energy consumption. In this respect, research is particularly dedicated to the optimisation of the distillation unit, with the relevant dimensioning of double or triple effect columns, with the mechanical or thermal compression of vapours, which represent a real advantage due to the low energy consumptions. The same is true for the evaporation of the effluents coming from the column bottom: multi-effect evaporators, with mechanical and/or thermal compression undoubtedly allow a substantial energy saving. Nevertheless, the energy consumed for drying the non-fermentable residues after distillation (DDG) is not taken into due consideration.

The plant section relating to this part of the process is normally considered as being of secondary importance in the distillation field: this is partially due to the fact that not all distilleries must dry this product (which, in many cases, is sold in the humid state directly to agricultural farms), and, above all, because the technologies currently used are either very simple but expensive from an energy point of view (3.8 MJ/kg evaporated water), or are thermally effective (2.7 MJ/kg evaporated water) but complex and costly, therefore the investment can hardly be justified.

A solution for drying the distillation residues is provided in the description of modem technologies for the production of alcohol, as mentioned in the "The alcohol textbook, 3^{rd} edition, 1999", by the University of Nottingham (UK), wherein, on page 266 simple vapour drying is illustrated, as such however not reusable in other steps of the preparation process. The same text, on page 259, underlines the recycling of the effluents from the bottom of the distillation columns in the formation of the mix to be hydrolyzed, and this requires the use of chemicals for neutralization. Finally, an important characterizing detail of the known processes for the preparation of alcohol is that a satisfactory plant efficiency is obtained when the total energy used is equal to 39,000 BTM and 1.15 KWH per gallon, which correspond to the Italian measures of 2580 Kcal/liter and 0.26 KWH/liter, equal to a total of 1175 MJ/HN (see page 267).

EP-A-0 237520 teaches a process for the preparation of ethanol by fermentation of sugar-containing raw materials, in which at least part of the water-rich distillation residue is recirculated to the raw materials, in order to reduce the consumption of process water and energy, as well as the effluent problem.

The Applicant has now found that it is possible to produce ethanol starting from cereals, according to the best transformation yields and obtaining the best quality currently on the market, through a process, object of the present invention, which, based, among other expedients, on the use of a particular drying system of the non-fermentable residues, allows an overall specific energy consumption lower than 700 MJ/HN, at the same time, avoiding other drawbacks which would appear inevitable by operating according to the above-mentioned technologies of the known art.

An object of the present invention relates to an improved process for the production of ethanol from cereals, which comprises the following fundamental operations:
- grinding of the cereal,
- mixing of the ground product with the condensed products returning from the process, only,
- hydrolysis of the mix thus obtained,
- mixing of the hydrolyzed fluid mix with distillation residues, so-called borlande,
- saccharification of the mix thus diluted and subsequent fermentation,
- distillation for the separation of the alcoholic phase from the borlande containing all the non-fermentable residues,
- clarification and separation of the borlande constituents,
- recycling of part of the clarified borlande to the saccharification step,
- sending of the cake obtained from the centrifuged borlande to the drying step,
- mixing of the above with a concentrate coming from the evaporation of the clear borlande portion not recycled to saccharification,
- drying of the distillation residue mix thus obtained by means of saturation with overheated vapour,
- vapour distribution between a recycled vapour to the drier and a motor vapour for supplying driving power to the process units,
- recycling of condensates to the grinding product of the cereal.

The process described above allows the following main advantages to be obtained with respect to the technologies currently in use for the preparation of ethanol:
- total reuse of the thermal energy by exploiting the same energy in the various plant units,
- production of a volume of process condensates sufficient for effecting the first starch dilution, without having to resort to fresh water or borlande,
- reduction in the use of chemicals (enzymes, acids and bases) necessary for controlling the process reactions, thanks to the high concentration of dry product, recycling of condensates and borlande in different process steps,
- optimum balance of thermal and electrical energy obtained from the combustion of gas,
- 90% reduction in emissions to the atmosphere and liquid waste.

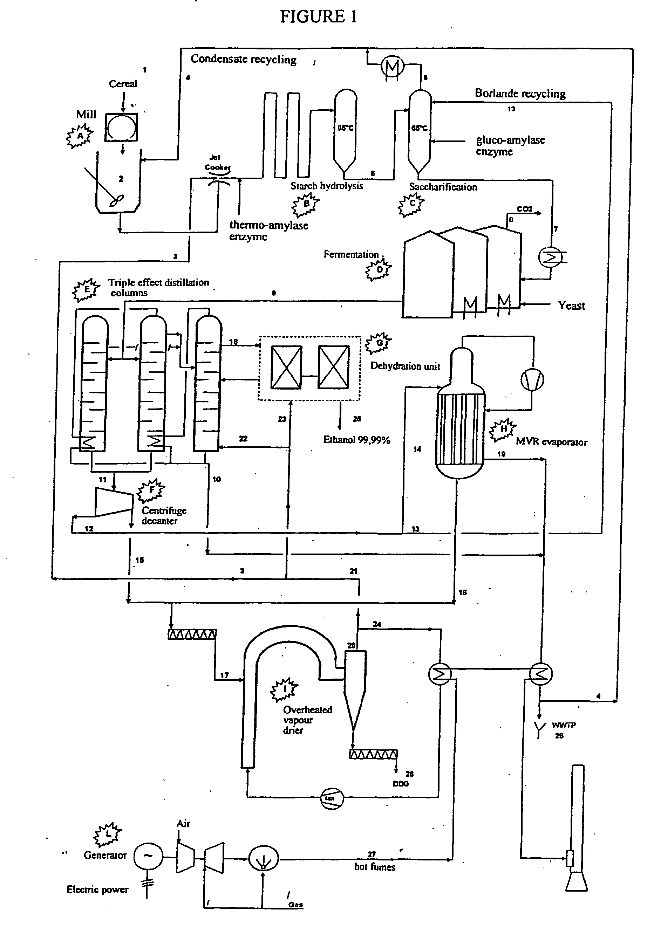

The process for the production of ethanol according to the present invention is further explained in detail in the scheme shown in fig. 1, which describes the process production steps with the material and energy flows.

The scheme is provided for purely illustrative purposes, variations in the plant details obviously being possible, also with reference to the type of cereal used, without limiting the objective of the present invention; again for purely illustrative purposes, the following description is provided, assuming wheat as reference cereal.

With reference to the scheme shown in the figure, the cereal (1) destined for the production of ethanol, is sent, after adequate cleaning, from the storage silos to the grinding unit (A).

Said unit consists of a hammer mill having a fine mesh grid so as to guarantee a particle size of the pulverized product of about 2-3 mm max.

A particle size which is too fine creates problems in the fiber separation step, if it is too coarse, problems can arise during the starch hydrolysis.

The product ground in the mill is then mixed with the condensates (4) returning from the process, only. These products, before being reused, are heated to a temperature of 90-95°C through recovery of the heat still present in the exhausted fumes of the combusted gas.

The mixture (2) obtained has a dry content ranging from 35 to 43%, a temperature of about 60-65°C and a pH of 5.5-5.7: these are ideal parameters for the subsequent starch hydrolysis unit (B). The hydrolysis reaction takes place by heating the whole mixture to a temperature of 92-105°C (the temperature changes according to the cereal type and residence times) with an emission of fresh vapour (3) and the addition of the suitable enzyme (thermo-amylases).

The flow rate of the necessary vapour ranges from 15 to 25 KG/HN in relation to the dry content and the temperature to be reached.

Lengthy hydrolysis times and a high concentration of the dry matter in the mixture, which are envisaged in the present project, are parameters which have a positive influence on the enzyme consumptions, which' are reduced.

The fluid mixture of hydrolyzed starch (5) sent to the Saccharification (C) and Fermentation(D) Units is further diluted, this time with the distillation residues, borlande (13), until a total dry content equal to about 25-28%, is reached.

A fraction of this dry product, within the range of 13-15%, consists of fermentable sugars.

The use of clarified borlanda has two important advantages:
- it reduces the volume of effluents to be sent to the final evaporation. If the borlanda is not reused, in fact, it is an effluent which must be evaporated for the recovery of the dry content;
- the fact that the borlanda has a strongly acid pH, allows a saving in the usc of the acid necessary for bringing the must under fermentation to values of 3.2-3.6.

The mixture (7) is cooled to 65°C using the flash system, to prevent damaging the new saccharification enzyme (gluco-amylases), contemporaneously with the dilution and addition of the enzymes, and then to 30°C by means of a water exchanger, before entering the fermentation tanks to reach the temperature suitable for alcoholic fermentation.

The two reactions are contemporaneous: as the dextrose is produced by saccharification, it is transformed by the yeast to alcohol and carbon dioxide by fermentation.

The fermentation is of the continuous type, it lasts about 70-80 hours and the temperature control keeps the temperature at values not higher than 35°C.

The yeast propagation is effected separately, and the yeast is continuously added to the inlet flow of the mixture to be fermented (must).

There are two fermentation products:
- carbon dioxide CO₂ (8) in gaseous form, which can be recovered by refinement and compression,
- ethanol contained in the wine (9) in an amount equal to about 12%.
   The percentage of ethanol contained in wine can vary considerably in relation to the sugar content of the starting must. When high degrees are obtained, there is a reduction in the volumes for the same amount of ethanol produced, which contributes to energy saving and allows the use of equipment having reduced dimensions.

The Distillation Unit (E) receives the wine (9) and effects the separation of the alcoholic phase from all the other substances by means of a tray-column system.

An azeotropic alcoholic mix is concentrated in the head: ethanol 96% and water 4% (18).

In this particular case there are three columns: a rectifying column, a distillation column at atmospheric pressure and a vacuum column.

The system is suitable for energy saving: the vapour (22) under a pressure of 2.6-3 bar, coming from the drier and purified, heats the rectifying column which, in turn, heats the atmospheric column and, downstream, the vacuum column. The vapour consumption per hectolitre of ethanol (HN) can be reduced to 110 kg/HN.

The borlande (11) present at the bottom of the distillation columns, which contain all the non-fermentable residues, are sent to the Horizontal Centrifuge Decanters (F) for the clarification and separation of the insoluble substances and fibers.

These decanters divide the flow into two parts: one part called cake (1\5) to be dried, the other part being the clarified borlanda (12).

The alcoholic azeotropic mix (18) is extracted at the head of the rectifying column, and is sent to the Ethanol Dehydration Unit (G).

The consolidated molecular sieve technology is used for drying the alcohol from 96% to 99.99%. Two reactors in parallel and used alternately, effect drying cycles with water adsorption on zeolite inert matter. At the end of each cycle, the resins are regenerated by means of vapour (23).

The vapour consumption for the dehydration proves to be 55 Kg/HN.

A part of the clarified borlande (14) which has not been recycled, is destined for evaporation and is then sent for final concentration in the MVR Evaporation Unit (H).

The concentration system is of the tube-bundle type with forced circulation of the liquid phase and Mechanical Vapour Recompression: this solution was adopted for optimising the use of the electric energy produced by the gas turbine, at the same time saving thermal energy.

The thermal consumption is null, in fact, and the electric consumption is equal to 20 KWH/T of evaporated water.

The final concentrate (16), having a dry matter of about 27-30%, is mixed with the cake (15) coming from the horizontal decanters and sent (17) to the Overheated Vapour Drying Unit (I).

The drying process is effected by saturation of the overheated vapour (water is transferred from the humid mixture to vapour, making it saturated) (20), part of this is recycled vapour (24) fected by a fan after heating (the cycle is then repeated) and the other part is motor vapour (21) which, as it is at a pressure of 3 bar, can give driving power to the process units.

In particular, the vapour (21) has three uses:
- vapour (23) to the dehydration molecular sieves: 55 Kg/HN,
- vapour (22) to the column distillation unit: 110 Kg/HN,
- vapour (3) to the starch hydrolysis unit: 15-25 Kg/HN.

The only external energy source for the proposed plant is the gas which feeds the electric power turbo-generator.

The production is equal to 33 KWH/HN, an amount sufficient for covering the electric demand of the plant.

All the thermal energy necessary for the production of ethanol, 200 Kg vapour/HN, is obtained from the hot fumes at the outlet of the turbo-gas.

The overall process, according to the scheme indicated, effects the complete transformation of the cereal into ethanol 99.99% and the drying of the non-fermentable residues with an overall specific consumption of power lower than 700 MJ/HN.

With specific reference to the example, the advantages which can be obtained by producing ethanol according to the process of the present invention, can be explained in more detail.

### As far as energy is concerned:

a. production of a hectolitre of ethanol (HN) with gas consumption as the only energy source to the overall plant, equal to 21 NM3/HN - 700 MJ/HN. This value represents a minimum with respect to the technologies currently used. It is obtained by reusing the thermal power of the DDG drying and by balancing the use of electric and thermal power obtained by means of suitable technological and plant engineering selections (double effect distillery, effluent evaporation with mechanical compression of the vapours). The comburent gas feeds the turbine where all the electric power necessary for the running of the plant is produced, 33 KWH/HN. The residual thermal power is contained in the hot fumes at the turbine outlet. This thermal power represents the only heat source for running the whole distillery. In practice, the hot fumes at the outlet of the turbo are sent to the DDG drying plant, where they overheat the drying medium which, in this unit is not air but vapour. A saturated vapour is available at the end of the drying phase, which has such flow rate (180-200 kg/HN) and pressure (3 bar) characteristics as to allow it to be used in the other process units.
b. Starch hydrolysis at a high concentration of dry matter (35-43%): this allows a considerable saving, as both the heating power and the heat to be removed at the end of the reaction are halved.
c. Starch hydrolysis: this uses the condensates collected during the various steps of the production process as the only diluent. This is possible also thanks to the drying system which allows the recovery of its condensates, otherwise the necessary volume would not be available.

### With respect to the environment:

The normal drying procedures produce hot, humid air, with amounts of polluting substances which must be continuously monitored. The use of overheated vapour as drying medium not only represents a considerable thermal saving, but also a great environmental advantage as it reduces the emissions to the atmosphere to practically zero, due to the drying. Another considerable advantage of this process consists of the almost total absence of effluents, as it includes a wide reuse of residual water. The discharge of effluents (26) to the effluent treatment unit is extremely limited compared to a standard ethanol plant, with a great advantage for the environment, from an energy point of view and a reduced investment for the purification unit (WWTP).

### As far as the ingredients are concerned:

The process also has considerable advantages with respect to the ingredients, as the solutions suggested decidedly reduce the use of enzymes and chemicals (acids and bases) necessary for the reaction control.

### Enzymes:

The use of enzymes is reduced by effecting the transformation in doubled times, with strong shaking to increase the number of contacts and with higher concentrations of dry matter.

The longer reaction times and vigorous shaking considerably reduce the amount of enzymes required.

Higher concentrations of dry matter reduce the overall volume: as a result, there is a lower dispersion of enzymes and consequently an enhanced efficiency.

### Acids and bases:

The use of chemicals, which would be very high in the presence of the recycling of borlande, is reduced by an expedient which is part of the novelty of the process: the first starch dilution, before its hydrolysis, is effected exclusively with condensates, without the reuse of borlande. This is a great advantage in that, as the hydrolysis reaction requires an optimal pH not lower than 5.5-5.7, the necessity for a chemical Base (such as Soda) to neutralise the strong acidity of the borlande, would be extremely high if, as normally happens, borlande are used for the dilution of starch; when, on the contrary, water is used for the dilution, acid is not necessary, the energy costs however are considerably increased.

Furthermore, the subsequent saccharification and fermentation reaction must take place in an acidic environment, and consequently, after neutralization (when borlande are used) a re-acidification would be necessary to pH values around 3.2-3.6, this time using a high amount of acid.

The present process envisages that, before hydrolysis, only the condensates be used, as diluent recycled from the process, whose neutralization is easy and not onerous, whereas, after hydrolysis, the subsequent dilution is effected with the use of recycled clear borlande.

As these are acid, they allow the pH to be lowered, practically without the use of acids.

## Claims

1. An improved process for the preparation of ethanol from cereals comprising the following fundamental operations:
- grinding of the cereal,
- mixing of the ground product with the condensed products returning from the process, alone,
- hydrolysis of the mix thus obtained,
- mixing of the hydrolyzed fluid mix with distillation residues,
- saccharification of the mix thus diluted and subsequent fermentation,
- distillation for the separation of the alcoholic phase from the borlande containing all the non-fermentable residues,
- clarification and separation of the borlande constituents,
- recycling of part of the clarified borlande to the saccharification step,
- sending of the borlande cake to the drying step,
- mixing of the above with a concentrate coming from the evaporation of the clear borlande portion not recycled to saccharification,
- drying of the distillation residue mix thus obtained by means of saturation with overheated vapour,
- vapour distribution between a recycled vapour to the drying section and a motor vapour for supplying driving power to the process units,
- recycling of the condensates to the grinding product of the cereal.

2. The improved process for the production of ethanol from wheat according to the previous claim, wherein the condensates returning from the process are the only diluent of the ground product, and they are heated to a temperature ranging from 90 to 95°C, before mixing with the ground cereal.

3. The improved process for the production of ethanol from cereals according to claim 1, wherein borlande are used for the dilution but only after the hydrolysis reaction, and the mixture of the hydrolysed product is diluted with the borlande until an overall dry product of about 25-28% is obtained.

4. The improved process for the production of ethanol from cereals according to claim 1, wherein the drying process of the distillation residues is performed by saturation of the overheated vapour obtained by means of water transfer from the humid mix to vapour.

5. The improved process for the production of ethanol from cereals according to claim 1 and claim 4, wherein the overheated vapour is divided into recycled vapour to the drier and motor vapour for the process units.

6. The improved process for the production of ethanol according to the previous claim, wherein the motor vapour contributes to the supply of motor power preferably for:
- the dehydration molecular sieves
- the distillation unit
- the starch hydrolysis unit.

7. The improved process for the production of ethanol according to claim 1, wherein the specific power consumption supplied by the gas which feeds the electric power turbo-generator, is reduced to 700 MJ/HN.

8. The improved process for the production of ethanol according to claim 1, wherein the dilution of the ground product is effected in different points (after hydrolysis and saccharification) and with different diluents (condensate first and borlande after) so as to obtain a considerable saving of the ingredients for the pH control and a remarkable energy saving due to the reduced volume to be heated and subsequently cooled down.

9. The improved process for the production of ethanol according to claim 1, wherein the process discharges are reduced by 80%.

## Patentansprüche

1. Verbessertes Verfahren für die Herstellung von Ethanol aus Getreiden, umfassend die folgenden grundlegenden Operationen:
- Mahlen des Getreides;
- Mischen des gemahlenen Produkts mit den aus dem Verfahren zurückerhaltenen kondensierten Produkten allein;
- Hydrolyse der so erhaltenen Mischung;
- Mischen der hydrolysierten Fluidmischung mit Destillationsresten;
- Saccharifikation der so verdünnten Mischung und anschließende Fermentation;
- Destillation für die Abtrennung der alkoholischen Phase von der alle nicht fermentierten Reste enthaltenden Borlande;
- Klarifikation und Abtrennung der Borlande-Bestandteile;
- Rückführen eines Teils der klarifizierten Borlande in den Saccharifikationsschritt;
- Überführen des Borlande-Kuchens zu dem Trocknungsschritt;
- Mischen des oben Genannten mit einem Konzentrat, das aus der Abdampfung des nicht zur Saccharifikation rückgeführten klaren Borlande-Anteils stammt;
- Trocknen der so erhaltenen Destillationsrestmischung mittels Saturation mit überhitztem Dampf;
- Dampfverteilung zwischen einem in den Trocknungsabschnitt rückgeführten Dampf und einem Motordampf für die Zuführung von Antriebskraft zu den Prozesseinheiten;
- Rückführen der Kondensate zu dem Mahlprodukt des Getreides.

2. Verbessertes Verfahren für die Herstellung von Ethanol aus Weizen gemäß dem vorhergehenden Anspruch, wobei die aus dem Verfahren zurückerhaltenen Kondensate das einzige Verdünnungsmittel des gemahlenen Produkts sind und diese auf eine Temperatur im Bereich von 90 bis 95°C vor dem Mischen mit dem gemahlenen Getreide erwärmt werden.

3. Verbessertes Verfahren für die Herstellung von Ethanol aus Getreiden gemäß Anspruch 1, wobei Borlande für die Verdünnung verwendet werden, jedoch erst nach der Hydrolysereaktion, und die Mischung des hydrolysierten Produkts mit der Borlande verdünnt wird, bis ein Gesamttrockenprodukt von etwa 25-28 % erhalten wird.

4. Verbessertes Verfahren für die Herstellung von Ethanol aus Getreiden gemäß Anspruch 1, wobei das Trocknungsverfahren der Destillationsreste durch Saturation des überhitzten Dampfes erfolgt, welcher durch den Wasserübergang von der feuchten Mischung in Dampf erhalten wird.

5. Verbessertes Verfahren für die Herstellung von Ethanol aus Getreiden gemäß Anspruch 1 und Anspruch 4, wobei der überhitzte Dampf in rückgeführten Dampf zu dem Trockner und Motordampf für die Prozesseinheiten aufgeteilt wird.

6. Verbessertes Verfahren für die Herstellung von Ethanol gemäß dem vorhergehenden Anspruch, wobei der Motordampf zu der Bereitstellung von Motorleistung beiträgt, vorzugsweise für:
- die Entwässerungs-Molekularsiebe
- die Destillationseinheit
- die Stärke-Hydrolyseeinheit.

7. Verbessertes Verfahren für die Herstellung von Ethanol gemäß Anspruch 1, wobei der spezifische Energieverbrauch, bereitgestellt durch das Gas, welches den mit elektrischer Energie betriebenen Turbogenerator speist, auf 700 MJ/HN verringert wird.

8. Verbessertes Verfahren für die Herstellung von Ethanol gemäß Anspruch 1, wobei die Verdünnung des gemahlenen Produkts an verschiedenen Punkten (nach der Hydrolyse und Saccharifikation) und mit verschiedenen Verdünnungsmitteln (Kondensat zuerst und Borlande danach) erfolgt, um eine beträchtliche Einsparung bei den Bestandteilen für die pH-Kontrolle und eine nennenswerte Energieersparnis aufgrund des reduzierten Volumens, das zu erwärmen und anschließend abzukühlen ist, zu erzielen.

9. Verbessertes Verfahren für die Herstellung von Ethanol gemäß Anspruch 1, wobei die Prozessabflüsse um 80 % reduziert werden.

## Revendications

1. Procédé amélioré pour la préparation d'éthanol à partir de céréales comprenant les opérations fondamentales suivantes :
. broyage de la céréale,
. mélange du produit broyé uniquement avec les produits condensés provenant du processus,
. hydrolyse du mélange ainsi obtenu,
. mélange du mélange fluide hydrolysé avec les résidus de distillation,
. saccharification du mélange ainsi dilué et fermentation postérieure,
. distillation pour la séparation de la phase alcoolique des huiles de fusel contenant tous les résidus non-fermentiscibles, clarification et séparation des constituants des huiles de fusel,
. réutilisation de la partie clarifiée des huiles de fusel l'étape de saccharification,
. envoi du gâteau d'huiles de fusel à l'étape de séchage,
. mélange de ce qui précède avec un concentré provenant de l'évaporation de la partie claire des huiles de fusel non réutilisée à la saccharification,
. séchage du mélange de résidu de distillation ainsi obtenu par saturation avec de la vapeur surchauffée,
. distribution de vapeur entre une vapeur réutilisée vers la section de séchage et une vapeur motrice pour alimenter en énergie d'entraînement les unités du procédé,
. réutilisation des condensats pour le produit de broyage de la céréale.

2. Procédé amélioré pour la production d'éthanol à partir de blé selon la revendication précédente, dans lequel les condensats provenant du procédé constituent le seul diluant du produit broyé, et sont chauffés à une température comprise entre 90 et 95°C, avant mélange avec la céréale broyée.

3. Procédé amélioré pour la production d'éthanol à partir de céréales selon la revendication 1, dans lequel les huiles de fusel sont utilisées pour la dilution mais seulement après la réaction d'hydrolyse, et le mélange du produit hydrolysé est dilué avec les huiles de fusel jusqu'à ce qu'un produit sec global d'environ 25 à 28% soit obtenu.

4. Procédé amélioré pour la production d'éthanol à partir de céréales selon la revendication 1, dans lequel le procédé de séchage des résidus de distillation est effectué par saturation de la vapeur surchauffée obtenue au moyen de transfert de l'eau du mélange humide à la vapeur.

5. Procédé amélioré pour la production d'éthanol à partir de céréales selon la revendication 1 et la revendication 4, dans lequel la vapeur surchauffée est divisée en vapeur réutilisée vers le sécheur et en vapeur motrice pour les unités du procédé.

6. Procédé amélioré pour la production d'éthanol selon la revendication précédente, dans lequel la vapeur motrice contribue à fournir de l'énergie motrice préférentiellement pour :
. les cribles moléculaires de déshydratation
. l'unité de distillation
. l'unité d'hydrolyse d'amidon.

7. Procédé amélioré pour la production d'éthanol selon la revendication 1, dans lequel la consommation d'énergie spécifique fournie par le gaz qui alimente le turbogénérateur à énergie électrique, est réduite à 700 MJ/HN.

8. Procédé amélioré pour la production d'éthanol selon la revendication 1, dans lequel la dilution du produit broyé est effectuée en différents points (après hydrolyse et saccharification) et avec différents diluants (condensats d'abord et huiles de fusel ensuite) afin d'obtenir une économie considérable des ingrédients pour le contrôle du pH et pour effectuer une économie remarquable d'énergie due au volume réduit à chauffer et ensuite à refroidir.

9. Procédé amélioré pour la production d'éthanol selon la revendication 1, dans lequel les déchets du procédé sont réduits de 80%.
